# EUROPEAN PATENT SPECIFICATION

(11) **EP 2 111 788 B1**
(45) Date of publication and mention of the grant of the patent: **08.01.2014**
(21) Application number: 08710574.8
(22) Date of filing: 23.01.2008
(51) Int. Cl.: A61B 5/07, A61B 1/00, A61B 1/273, A61B 1/04

(54) **DEVICE FOR CHECKING FOR LUMEN PASSAGE**
VORRICHTUNG ZUR PRÜFUNG AUF LUMEN-PASSAGEN
DISPOSITIF PERMETTANT DE CONTRÔLER LE PASSAGE D'UNE LUMIÈRE

(30) Priority: 30.01.2007 JP 2007019910; 30.01.2007 JP 2007019912
(43) Date of publication of application: 28.10.2009
(73) Proprietor: Olympus Medical Systems Corp., Tokyo 151-0072 (JP)
(72) Inventor: KAWANO, Hironao, Shibuya-ku Tokyo 151-0072 (JP); YOKOI, Takeshi, Shibuya-ku Tokyo 151-0072 (JP)
(74) Representative: von Hellfeld, Axel
(86) International application number: PCT/JP2008/050888
(87) International publication number: WO 2008/093578

(56) References cited:
- EP-A1- 1 685 797
- WO-A1-2005/087079
- WO-A2-03/005877
- JP-A- 2004 248 956
- JP-A- 2005 508 668
- JP-A- 2006 130 240
- JP-A- 2006 142 013
- JP-A- 2006 239 439
- JP-A- 2006 280 940

## Description

### TECHNICAL FIELD

The present invention relates to a lumen passability checking device that is inserted into a body of a subject before a capsule medical device such as a capsule endoscope is actually used in order to check beforehand whether the capsule medical device can pass through a lumen and an organ such as a small intestine, and also relates to methods of dissolving and manufacturing the device.

### BACKGROUND ART

Capsule medical devices such as capsule endoscopes that pass through lumens and organs of a subject to perform observations, examinations, or treatments have been proposed and put to practical use. There is a problem in using such a capsule medical device in that when there is an abnormality such as a stenosis part at a relatively narrow lumen or organ such as a small intestine, a swallowed capsule medical device stagnates in the vicinity of the stenosis part in the lumen.

In view of the problem, a lumen passability checking capsule (a pretest capsule for endoscope) has been proposed which has the same size and shape as an actual capsule medical device and is inserted into a body of a subject in order to check beforehand whether lumens include a portion such as a stenosis part at which the capsule medical device may stagnate for a long time (see, e.g., Patent Documents 1 to 4). When such a lumen passability checking capsule is normally excreted from the body, it is determined that there is no abnormality such as stenosis and thus the application of a capsule endoscope is possible. When the lumen passability checking capsule is not normally excreted from the body, it is determined that there is an abnormality such as a stenosis and therefore the application of a capsule endoscope is unsuitable. Such a lumen passability checking capsule needs to maintain its capsule shape for a predetermined time or more in order to check whether a passage failure occurs due to a stenosis and also needs to dissolve or decompose after the predetermined time so that the lumen passability checking capsule does not stagnate at the stenosis part in the body and is excreted. In order to comply with such a request, various materials such as enteric materials including alkali-soluble materials such as (acetate/succinate) hydroxypropyl methylcellulose, which dissolves in the intestines but not in the stomach, have been used as the material for forming lumen passability checking capsules. As enteric materials, natural polysaccharide and polyalcohol compositions are also known (see, e.g., Patent Documents 5 and 6).
EP 1 685 797 A1 discloses a device for enabling an examining device to pass through an abnormally configured body lumen. The device passes through a body lumen, while maintaining an initial dimension. Upon encounter with an abnormally configured body lumen, the device is depleted and takes on its final dimensions after a predetermined time period. A coating has initial dimensions that are changeable to final dimensions and a bolus comprises particles that are of final dimension.
WO 03/005877 A1 refers to a device for examining a body lumen having two operational phases, namely an initial phase, in which the device is of initial dimension, and a final phase, in which the device is of final dimension. In the initial phase the device can pass freely through an normally configured body lumen. In the final phase, the device can pass freely through a body lumen, even if it is abnormally configured.

Patent Document 1: Published Japanese translation of a PCT application No. 2005-508668
Patent Document 2: Japanese Patent Application Laid-Open No. 2004-248956
Patent Document 3: US Patent Application Laid-Open No. 2005/0063906
Patent Document 4: Japanese Patent Application Laid-Open No. 2006-142013
Patent Document 5: Japanese Patent Application Laid-Open No. H3-2328815
Patent Document 6: Japanese Patent Application Laid-Open No. H11-49668

### DISCLOSURE OF INVENTION

### PROBLEM TO BE SOLVED BY THE INVENTION

However, Patent Document 1 does not mention the ease of crushing the capsule shape in the configuration made of an enteric material after elapsing a predetermined time, and there occurs a case in which the excretion after elapsing the predetermined time is not reliably performed when there is a stenosis part.

Further, although a complete dissolution time (for which the capsule shape is maintained) of a lumen passability checking capsule is desired to set at one hundred hours or longer, or in a range between one and seven days, controlling the complete dissolution time by controlling the material depending on the enteric material is difficult. When using the enteric material disclosed in the Patent Documents, the complete dissolution time is about one day, at the very most, in view of the size of the lumen passability checking capsule. Therefore, the desired complete dissolution time cannot be secured. A body-passing time period of a lumen passability checking capsule for a healthy person is often two or three days, and thus it cannot secure a certain time period needed for checking the passability irrespective of persons and cannot check the passability reliably.

The present invention is made in view of the above problems. An object of the present invention is to provide a lumen passability checking device with a simple structure of which capsule shape easily crushes after a predetermined time in a lumen so that the lumen passability checking device is reliably excreted and to provide methods of dissolving and manufacturing the device.

Further, an object of the present invention is to provide a lumen passability checking device that reliably maintains its capsule shape for a predetermined time needed for checking the passability.

### MEANS FOR SOLVING PROBLEM

A lumen passability checking device according to an aspect of the present invention is defined by independent claim 1. Specific embodiments of the lumen passability checking device are defined by the dependent claims.

### EFFECT OF THE INVENTION

In the lumen passability checking device in accordance with an aspect of the present invention, since the insoluble unit is formed of the thin film covering the surface of the soluble unit, which forms the capsule-shaped structure, and exposing a part of the surface as an opening, the insoluble unit formed of the thin film cannot maintain the capsule shape and collapses when the soluble unit dissolves in the lumen after elapsing the predetermined time. Therefore, the excretability is reliably increased with the simple structure.

In the lumen passability checking device in accordance with an aspect of the present invention, since the insoluble unit is formed of the multiple thin films, even when the outermost layer of the insoluble unit is damaged by a pinhole, for example, the capsule shape is maintained by preventing the soluble unit from dissolving through the pinhole. Therefore, shortening of a time for which the capsule shape can be maintained is prevented.

In the lumen passability checking device in accordance with an aspect of the present invention, the second insoluble unit divides the inside of the soluble unit and forms the dissolution pathway that extends from the part of the surface to the inside. Thus, by lengthening the dissolution pathway, the dissolution time of the soluble unit in the lumen can be structurally extended. Therefore, the capsule shape can be maintained for a long time. The capsule shape can be reliably maintained for the period of time that is required for checking the passability, whereby the lumen passability can be reliably checked. Furthermore, when elapsing the predetermined time, the insoluble unit reliably collapses due to the dissolution of the soluble unit.

In the lumen passability checking device in accordance with an aspect of the present invention, the second insoluble unit forming the dissolution pathway is formed so as to extend toward the center of the capsule shape. Thus, the dissolution pathway can be formed which allows the dissolution to proceed from the center of the capsule shape to the outside, so that the time until when the soluble unit near the insoluble unit dissolves can be prolonged. Therefore, the capsule shape can be maintained for a long period of time.

In the lumen passability checking device in accordance with an aspect of the present invention, since the dissolution pathway is further divided by the third insoluble unit into smaller portions, the dissolution pathway is further lengthened and the capsule shape can be maintained for a longer period of time.

In an example of a lumen passability checking device, since the end of the second insoluble unit is connected to the end of the insoluble unit, the insoluble unit and the second insoluble unit are continuous on the outer surface of the capsule shape. Therefore, the capsule shape does not collapse from the end of the insoluble unit, and the capsule shape can be maintained for a long period of time.

In an example of a lumen passability checking device, the soluble unit is formed in the linear manner on the part of the insoluble unit forming the surface layer of the capsule shape such that the insoluble unit deforms to be small upon the dissolution of the soluble unit in the lumen. When the soluble unit dissolves in the lumen after the predetermined time, the soluble unit formed in the linear manner disappears, and the insoluble unit loses its rigidity and easily crushes to be small. Thus, even when the lumen passability checking device cannot pass through the lumens within the predetermined time and stagnates, the lumen passability checking device collapses after the predetermined time and is reliably excreted.

In an example of a lumen passability checking device, since the soluble unit is arranged in the linear manner at the convex parts at the both ends of the dome-shaped capsule shape, the convex parts having high rigidity lose its rigidity to be easily crushed when the soluble unit dissolves in the linear manner.

In an example of a lumen passability checking device, since the soluble unit is arranged in the linear manner in the longitudinal direction of the dome-shaped capsule shape, the insoluble unit forming the dome-shaped capsule shape is easily crushed in the radial direction when the soluble unit dissolves in the linear manner.

In an example of a lumen passability checking device, since the soluble unit is arranged in the linear manner in the circumferential direction around the longitudinal axis of the dome-shaped capsule shape, the insoluble unit forming the dome-shaped capsule shape is easily crushed in the axial direction when the soluble unit dissolves.

In an example of a lumen passability checking device, since the soluble unit is arranged in the spiral manner around the longitudinal axis of the dome-shaped capsule shape, the insoluble unit forming the dome-shaped capsule shape is easily crushed in the axial direction when the soluble unit dissolves.

In an example of a lumen passability checking device, the insoluble unit is formed of the plural members, which are joined together by the soluble unit. When the soluble unit dissolves, the insoluble unit is separated into smaller members, whereby the passability in the lumen is improved.

In an example of a lumen passability checking device, since the plural members forming the insoluble unit are sheet-like members, the insoluble unit becomes a sheet-like shape, loses its rigidity and easily curls to be small when the soluble unit dissolves.

In an example of a lumen passability checking device, since the sheet-like members are elastic members that curl due to their elasticity, the sheet-like members separated by the dissolution of the soluble unit automatically curl and reliably become smaller.

In an example of a lumen passability checking device, since the plural members forming the insoluble unit are tiny balls, the insoluble unit is separated into the tiny balls when the soluble unit dissolves. Thus, the lumen passability checking device reliably becomes smaller and even when one of the tiny balls drops off in the process, the capsule shape is maintained by the remaining tiny balls connected together by the soluble unit.

In an example of a lumen passability checking device, the second soluble unit that has higher solubility than the soluble unit is arranged in the insoluble unit. The second soluble unit is configured to dissolve after the dissolution of the soluble unit. After the soluble unit dissolves in the predetermined period of time, the second soluble unit dissolves in a short period of time. Therefore, the insoluble unit forming the capsule shape reliably collapses in a short period of time.

### BRIEF DESCRIPTION OF DRAWINGS

FIG. 1 is a schematic perspective view showing configuration of a lumen passability checking capsule.
FIG. 2 is a central vertical cross-sectional side view of what is shown in FIG. 1.
FIG. 3 is a schematic vertical cross-sectional side view showing a lumen passability checking capsule according to a first variation.
FIG. 4 is a schematic perspective view showing a lumen passability checking capsule according to a second variation.
FIG. 5 is a schematic perspective view showing a lumen passability checking capsule according to a third variation.
FIG. 6 is a side view of an end that is shown in FIG. 5.
FIG. 7 is a schematic perspective view showing a configuration of a lumen passability checking capsule.
FIG. 8 is a central vertical cross-sectional side view of what is shown in FIG. 7.
FIG. 9 is a schematic perspective view showing a lumen passability checking capsule according to a fourth variation.
FIG. 10 is a central vertical cross-sectional side view showing a lumen passability checking capsule according to a fifth variation, which defines a first embodiment of the present invention.
FIG. 11 is a cross-sectional view illustrating how a separated insoluble unit deforms.
FIG. 12 is a central vertical cross-sectional side view showing a lumen passability checking capsule according to a sixth variation.
FIG. 13 is a central vertical cross-sectional front view showing a configuration of a lumen passability checking capsule.
FIG. 14 is a partial cross-sectional view showing a lumen passability checking capsule according to a seventh variation.
FIG. 15 is a vertical cross-sectional front view showing a lumen passability checking capsule.
FIG. 16 is a vertical cross-sectional front view showing a lumen passability checking capsule according to an eighth variation.
FIG. 17 is a vertical cross-sectional front view showing an exemplary configuration of a lumen passability checking capsule according to a second embodiment of the present invention.
FIG. 18 is a side view of what is shown in FIG. 17.
FIG. 19 is a vertical cross-sectional front view showing an exemplary configuration of a lumen passability checking capsule according to a third embodiment of the present invention.
FIG. 20 is a vertical cross-sectional front view showing a lumen passability checking capsule according to a ninth variation.
FIG. 21 is a vertical cross-sectional front view showing an exemplary configuration of a lumen passability checking capsule according to a fourth embodiment of the present invention.
FIG. 22 is a central vertical cross-sectional side view showing an exemplary configuration of a lumen passability checking capsule according to a fifth embodiment of the present invention.
FIG. 23 is a front view of what is shown in FIG. 22.
FIG. 24 is a central vertical cross-sectional side view showing a lumen passability checking capsule according to a tenth variation.

### EXPLANATIONS OF LETTERS OR NUMERALS

- 11a, 11b: Convex part
- 12: Insoluble unit
- 12a, 12b: Sheet-like member
- 13: Soluble unit
- 14: Soluble unit
- 15, 15a, 15b: Detectable unit
- 16: Insoluble unit
- 16a: Sheet-like member
- 18: Insoluble unit
- 31: Insoluble unit
- 31a: Tiny ball
- 32: Soluble unit
- 41: Insoluble unit
- 43: Opening
- 61: Opening
- 62: Dissolution pathway
- 63: Insoluble unit
- 63a: End
- 64: Insoluble unit
- 81: Opening
- 82: Dissolution pathway
- 83: Insoluble unit

### BEST MODE(S) FOR CARRYING OUT THE INVENTION

Exemplary embodiments of a lumen passability checking device in accordance with the present invention, i.e., lumen passability checking capsules, are described with reference to the accompanying drawings. In the description below, the lumen passability checking capsules in the embodiments and variations thereof check the passability of, for example, the stomach and intestines as applicable lumens. The embodiments do not limit the scope of the present invention. In the accompanying drawings, the same components and corresponding components have the same numerals.

### (First example)

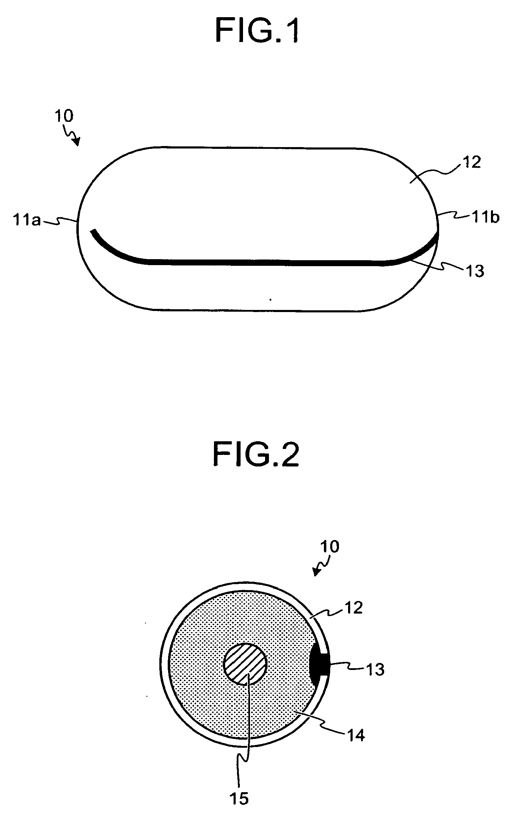

A lumen passability checking capsule in accordance with a first example of the present invention is described with reference to FIGS. 1 and 2. FIG. 1 is a schematic perspective view showing an exemplary configuration of a lumen passability checking capsule 10 in accordance with the first example. FIG. 2 is a central vertical cross-sectional side view of the lumen passability checking capsule 10.

The lumen passability checking capsule 10 in accordance with the first example schematically has substantially the same shape and size as a capsule endoscope used for examining and observing lumens such as the stomach and intestines and is insertable into a body of a subject. The lumen passability checking capsule 10 has a dome-shaped capsule shape that has convex parts 11a and 11b at both ends in a longitudinal direction thereof. Such the lumen passability checking capsule 10 having the dome-shaped capsule shape is configured by an insoluble unit 12, a soluble unit 13, a second soluble unit 14, and a detectable unit 15.

The insoluble unit 12 is made of a material that does not dissolve in the body and are not permeable (i.e., are not transmissive) to bodily fluids, such as a flexible metal and resin. The insoluble unit 12 is a thin-walled structure that forms a surface layer of the dome-shaped capsule shape excluding a portion and forms the outer shape of the dome-shaped capsule shape. The soluble unit 13 is made of a material that dissolves in the body and is arranged on a linear portion of the surface layer of the dome-shaped capsule shape so that, when the soluble unit 13 dissolves, the insoluble unit 12 deforms to be smaller. That is, a linear opening is formed on a part of the insoluble unit 12 so that the insoluble unit 12 cannot maintain its rigidity. The soluble unit 13 fills the linear opening so as to maintain the rigidity and to form a surface layer that maintains the dome-shaped capsule shape. In the first embodiment, the soluble unit 13 is arranged in a linear manner and extending between the convex parts 11a and 11b at the both ends in the longitudinal direction of the dome-shaped capsule shape. The soluble unit 13 not only fills the opening but also enters onto the inner wall side portion near the opening, so that it takes a predetermined time or more (e.g., one or two days or more) for the soluble unit 13 to dissolve.

The second soluble unit 14 is arranged inside the insoluble unit 12 forming the surface layer and forms the content of the lumen passability checking capsule 10 so that the second soluble unit 14 dissolves in lumens after the soluble unit 13 dissolves. The second soluble unit 14 is made of a material that has higher solubility than the soluble unit 13. In the first example, the soluble unit 13 is made of an enteric material that is hard to dissolve in the stomach but dissolves when travelling in the small intestine and the large intestine. For example, the alkali-soluble materials such as (acetate/succinate) hydroxypropyl methylcellulose disclosed in Patent Document 3, or natural polysaccharide and polyalcohol compositions obtained by homogeneously mixing at least one natural polysaccharide selected from the group consisting of carrageenan, algin acid, alginate, algin acid derivative, agar, locust bean gum, guar gum, amylopectin, pectin, xanthan gum, glucomannan, chitin, and pullulan in at least one selected from polyalcohol, sugar alcohol, monosaccharide, disaccharides, trisaccharides, and oligosaccharide disclosed in Patent Documents 4 and 5. On the other hand, the second soluble unit 14 is mainly made of a soluble material that easily dissolves such as natural polymer chitosan, gelatin, and cellulose.

The detectable unit 15 can be detected by a detecting apparatus that is located outside the body of the subject so as to confirm the intrabody-stagnating position or the current passing position of the lumen passability checking capsule 10. The detectable unit 15 can be a radiopaque member such as barium sulfate or metal (gold, titan, or stainless) or can be an electrical ID tag such as RF-ID (Radio_Frequency_Identification) tag. When the detectable unit 15 is a radiopaque member such as barium sulfate and metal, the detectable unit 15 is detected using an X-ray fluoroscope with which a user can see through the body of the subject in order to determine where the radiopaque member is so as to check whether the stagnation of the lumen passability checking capsule 10 is caused by the stenosis part. On the other hand, when the detectable unit 15 is the electrical ID tag such as RF-ID tag, the detectable unit 15 is detected using a tag reader-writer that transmits electricity to the electrical tag to activate IC chips so as to receive necessary information from the tag to recognize the existence of the tag. The detectable unit 15 itself is of a size that can pass through the stenosis part and is located in the second soluble unit 14 and in the center of the lumen passability checking capsule 10. The location of the detectable unit 15 is not necessarily limited to the center. When the insoluble unit 12 of the surface layer is made of a metal, the insoluble unit 12 itself can be used as the detectable unit 15, and the detectable unit 15 may not be provided in the second soluble unit 14.

Such the lumen passability checking capsule 10 is formed by inserting the detectable unit 15 into the insoluble unit 12 having the dome-shaped capsule shape and the opening formed therewith, filling the second soluble unit 14 therein, and filling the soluble unit 13 along the opening.

The thus configured lumen passability checking capsule 10 is ingested into the body of the subject a predetermined time (e.g., a few days) before examining and observing lumens by a capsule endoscope, whereby it can be checked beforehand whether the capsule endoscope can pass through the lumens such as the small intestine.

The lumen passability checking capsule 10 inserted into the body has the same size and shape as the corresponding capsule endoscope. Unless there is an abnormal portion at which a lumen is extremely narrowed by a stenosis part, for example, the lumen passability checking capsule 10 sequentially passes through the stomach, the small intestine, and the large intestine due to the peristalsis of the lumens and is eventually excreted from the body. Because the surface layer of the lumen passability checking capsule 10 is mostly made up of the insoluble unit 12, and the linear soluble unit 13 exposing to the outside as a part of the surface layer is made of the enteric material, the lumen passability checking capsule 10 advances without dissolving in the bodily fluid such as the gastric fluid until the lumen passability checking capsule 10 passes through the stomach. While the lumen passability checking capsule 10 advances through the small intestine and the large intestine, the soluble unit 13 made of the enteric material gradually dissolves. However, the lumen passability checking capsule 10 maintains its dome-shaped capsule shape for a predetermined period of time until the soluble unit 13 dissolves. Thus, when there is no abnormality such as a stenosis part in the small intestine and other lumens, the lumen passability checking capsule 10 advances without stagnating in the small intestine and the large intestine while maintaining the dome-shaped capsule shape and is excreted from the body within a predetermined period of time. In this case, it is confirmed that there is no abnormality such as stenosis part in the lumens and that the corresponding capsule endoscope can pass through without a problem.

On the other hand, when there is an abnormality such as a stenosis part in the small intestine or other lumens, the lumen passability checking capsule 10 is prevented from advancing in spite of the peristalsis and stagnates at the stenosis part. As the stagnation time elapses, the dissolution of the linear soluble unit 13 proceeds, and when a predetermined time elapses, the entire soluble unit 13 dissolves, and then the second soluble unit 14 is exposed to the outside and starts to dissolve. Because the second soluble unit 14 is made of a material that has higher solubility than the soluble unit 13, the second soluble unit 14 dissolves in a short period of time and flows out to the small intestine through the opening from which the soluble unit 13 has disappeared. Thus, in a short period of time, the insoluble unit 12 becomes hollow. Such the remaining insoluble unit 12 of the lumen passability checking capsule 10 has lost its rigidity for maintaining the dome-shaped capsule shape because the soluble unit 13 has disappeared from the linear opening and easily deforms to be small. By subjected to the peristaltic activity, the insoluble unit 12 is crushed and its outer diameter becomes smaller. In this manner, even when the lumen passability checking capsule 10 cannot pass through the small intestine within a predetermined time and stagnates at the stenosis part, the lumen passability checking capsule 10 reliably passes through the stenosis part and is excreted by being crushed after elapsing the predetermined time. Therefore, the lumen passability checking capsule 10 does not stay in the lumens. In the first embodiment, since the soluble unit 13 is arranged in the linear manner along the longitudinal direction of the dome-shaped capsule shape between the both ends in the longitudinal direction, after the soluble unit 13 dissolves, the insoluble unit 12 having the dome-shaped capsule shape easily crushes in the radial direction and the diameter of the front part thereof becomes smaller so that the passability through the stenosis part is improved.

Further, after elapsing the predetermined time, whether the lumen passability checking capsule 10 still stagnates in lumens or not can be checked by recognizing the existence of the detectable unit 15 using a detecting apparatus outside the body of the subject.

### (First variation)

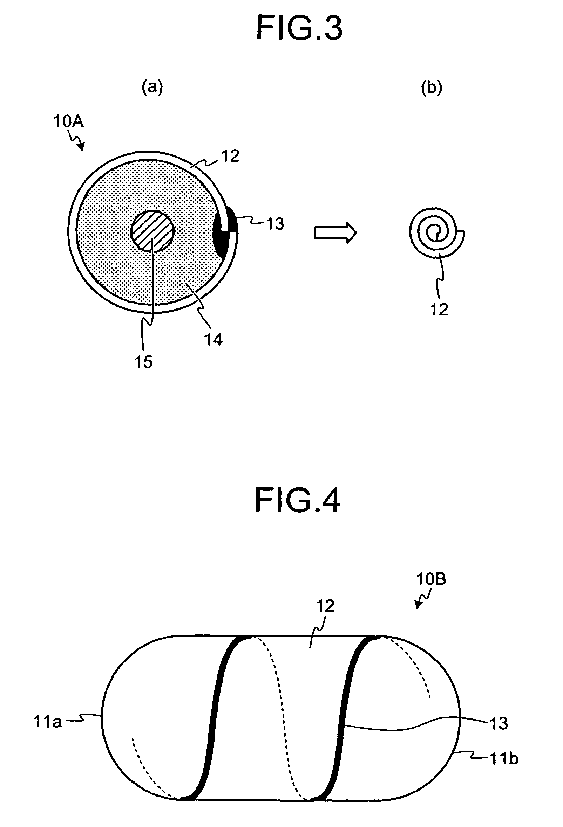

FIG. 3 is a schematic vertical cross-sectional side view showing a lumen passability checking capsule 10A in accordance with a first variation. In the lumen passability checking capsule 10A according to the first variation, the insoluble unit 12, which is the surface layer of the dome-shaped capsule shape, has elasticity. As shown in FIG. 3(a), both edges of the opening are arranged in a stepped manner without overlapping with each other in a radial direction and filled with the soluble unit 13. When the lumen passability checking capsule 10A in accordance with the first variation thus formed as shown in FIG. 3(a) in the same manner as FIG. 2 stagnates in the small intestine, the linear soluble unit 13 dissolves and then the inside second soluble unit 14 dissolves and flows to the outside so that the lumen passability checking capsule 10A becomes hollow. Then, the insoluble unit 12 of the lumen passability checking capsule 10A of which dome-shaped capsule shape has been maintained by the existence of the soluble unit 13 is forced to deform while curling and reducing its diameter due to the elasticity of the insoluble unit 12 as shown in FIG. 3(b), and thus easily passes through the stagnating point.

### (Second variation)

FIG. 4 is a schematic perspective view of a lumen passability checking capsule 10B in accordance with a second variation. In the lumen passability checking capsule 10B in accordance with the second variation, the soluble unit 13 is formed on the insoluble unit 12 in a spiral manner in a circumferential direction around the longitudinal axis of the dome-shaped capsule shape. When the lumen passability checking capsule 10B in accordance with the second variation stagnates in the small intestine, the spiral soluble unit 13 dissolves and then the inside second soluble unit 14 dissolves and flows to the outside so that the lumen passability checking capsule 10B becomes hollow. Then, the insoluble unit 12 of the lumen passability checking capsule 10B of which dome-shaped capsule shape has been maintained by the existence of the soluble unit 13 loses its rigidity and cannot maintain the dome-shaped capsule shape and collapses in a manner such that the insoluble unit 12 is broken into pieces along the axial direction, and thus easily passes through the stagnating point. A number of the spiral can be any suitable number. It is preferable that ends of the spiral line be extended to the ends of the convex parts 11a and 11b, respectively.

### (Third variation)

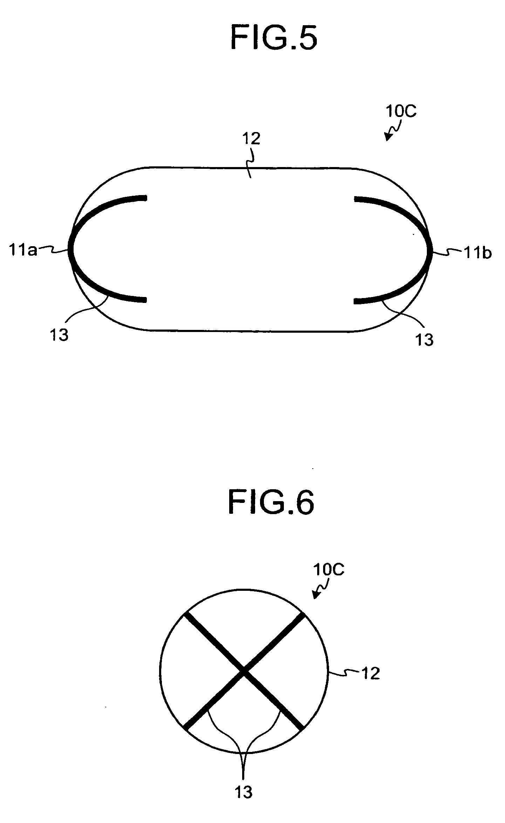

FIG. 5 is a schematic perspective view of a lumen passability checking capsule 10C in accordance with a third variation. FIG. 6 is a side view of an end of the lumen passability checking capsule 10C. In the lumen passability checking capsule 10C in accordance with the third variation, the soluble unit 13 is formed in an X-like shape by crossing lines on the convex parts 11a and 11b at the both ends of the insoluble unit 12 having the dome-shaped capsule shape. When the lumen passability checking capsule 10C in accordance with the third variation stagnates in the small intestine, the X-like shaped soluble unit 13 dissolves and then the inside second soluble unit 14 dissolves and flows to the outside so that the lumen passability checking capsule 10C becomes hollow. Then, the insoluble unit 12 of the lumen passability checking capsule 10C of which dome-shaped capsule shape has been maintained by the existence of the soluble unit 13 cannot maintain the dome-shaped capsule shape due to the reduction of rigidity in the convex parts 11a and 11b at both ends which used to have high rigidity. The insoluble unit 12 on the whole becomes a hollow cylinder and can be easily crushed, and thus easily passes through the stagnating point. The number of the lines crossing at the convex parts 11a and 11b is not limited to two. The number may be increased so that the lumen passability checking capsule 10C can be crushed more easily. Furthermore, the soluble unit 13 linearly formed at the convex parts 11a and 11b may be extended toward the center of the capsule along the longitudinal axis so that the lumen passability checking capsule 10C can be even more easily crushed.

### (Second example)

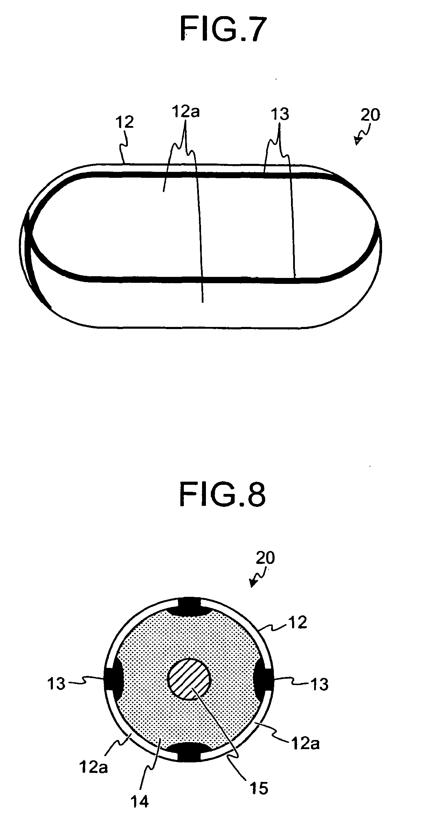

A lumen passability checking capsule in accordance with a second example of the present invention is described with reference to FIGS. 7 and 8. FIG. 7 is a schematic perspective view showing an exemplary configuration of a lumen passability checking capsule 20 in accordance with the second example. FIG. 8 is a central vertical cross-sectional side view of the lumen passability checking capsule 20.

The lumen passability checking capsule 20 in accordance with the second example has a substantially similar configuration to that of the lumen passability checking capsule 10 according to the first example. In the lumen passability checking capsule 20, two soluble units 13 formed linearly encircle the insoluble unit 12 along their lines so that the insoluble unit 12 is separated. The insoluble unit 12 is configured by plural members, e.g., four sheet-like members 12a which are joined together by the linear soluble unit 13. The soluble unit 13 is a combination of those in the first example and the third variation. The linear portion extending in the longitudinal direction and the linear portions at the convex parts 11a and 11b are continuous in the longitudinal direction to separate the insoluble unit 12. The soluble unit 13 allows the insoluble unit 12 to maintain its dome-shaped capsule shape.

When the lumen passability checking capsule 20 in accordance with the second example stagnates in the small intestine and the linear soluble unit 13 dissolves, the insoluble unit 12 of the lumen passability checking capsule 20 of which dome-shaped capsule shape has been maintained by the existence of the soluble unit 13 cannot maintain the dome-shaped capsule shape. The insoluble unit 12 is reliably separated into the sheet-like members 12a and deforms to be smaller, and thus easily passes through the stenosis part. The number of the lines of the soluble unit 13 is not limited to two. The number may be increased so that the insoluble unit 12 is separated into an increased number of smaller sheet-like members.

### (Fourth variation)

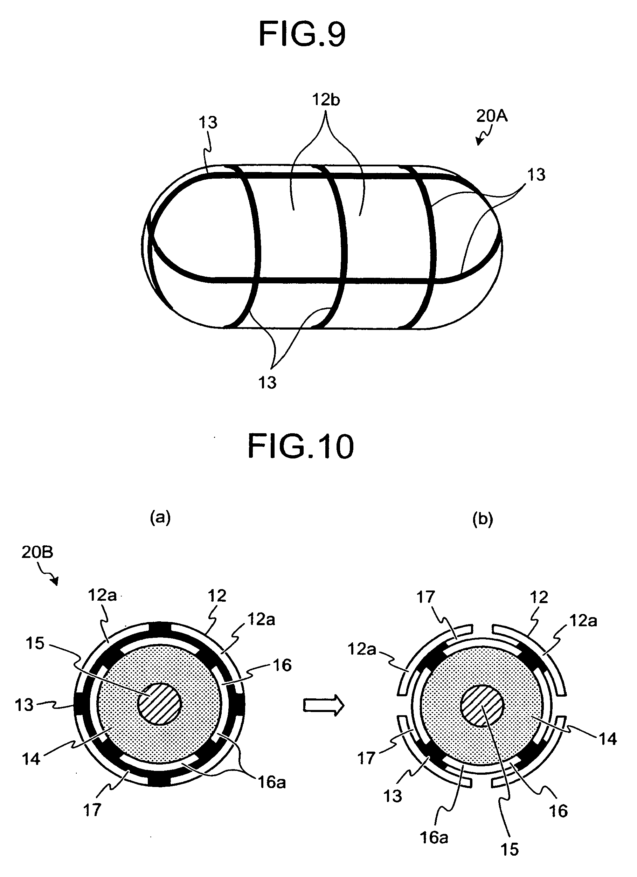

FIG. 9 is a schematic perspective view of a lumen passability checking capsule 20A in accordance with a fourth variation. In the lumen passability checking capsule 20A in accordance with the fourth variation, in addition to the linear soluble unit 13 encircling along the longitudinal direction, a linear soluble unit 13 that encircles in a circumferential direction is formed, so that the insoluble unit 12 is separated into an increased number of the sheet-like members 12b each being smaller. When the soluble unit 13 in the lumen passability checking capsule 20A in accordance with the fourth variation dissolves, the insoluble unit 12 is separated into the increased number of sheet-like members 12b each being smaller, whereby the insoluble unit 12 can more easily pass through the stagnating point. The number of lines of the soluble unit 13 may be any suitable number. The soluble unit 13 may be arranged obliquely relative to the longitudinal and circumferential directions, or may be combined with spiral ones.

### (Fifth variation which defining a first embodiment of the present invention)

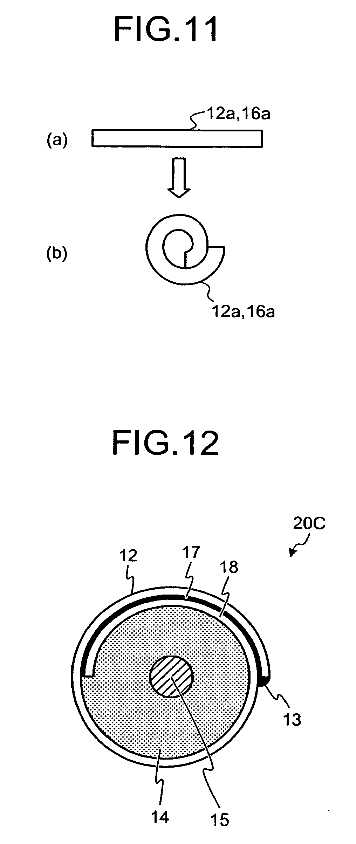

FIG. 10 is a central vertical cross-sectional side view of a lumen passability checking capsule 20B in accordance with a fifth variation. FIG. 11 is a cross-sectional view illustrating how an insoluble unit that is separated from the lumen passability checking capsule 20B deforms. As shown in FIG. 10(a), in the lumen passability checking capsule 20B in accordance with the fifth variation, a second insoluble unit 16 made of a material that does not dissolve in the body is formed in substantially parallel to and interior of the insoluble unit 12 via the soluble unit 13 in a layered manner. Thus, the insoluble unit has a two layered structure. In the same manner as shown in FIG. 7, openings that extends in the longitudinal direction and separates the second insoluble unit 16 into plural sheet-like members 16a are formed on a part of the second insoluble unit 16, and the openings are filled with the soluble unit 13 to join them together. The second insoluble unit 16 divides the soluble unit (i.e., the soluble unit 13 and the second soluble unit 14) and allows a dissolution pathway 17, which has advanced from a part (opening) of the surface toward the inside, to extend intermittently along the circumferential direction and to form cross-sectional arc shapes. The dividing positions (openings) in the insoluble unit 12 by the soluble unit 13 and the dividing positions (openings) in the second insoluble unit 16 by the soluble unit 13 are shifted in the circumferential direction around the longitudinal axis of the capsule-shaped capsule shape. In the fifth variation, the amount of shift is set to be equal so as to set the dissolution pathway 17 as long as possible. Sheet-like members 12a and 16a forming the insoluble units 12 and 16 are formed of elastic members that curl due to its elasticity.

When the lumen passability checking capsule 20B in accordance with the fifth variation stagnates in the small intestine and the linear soluble unit 13 of the surface layer dissolves, the dissolution of the soluble unit 13 advances from the surface to the inside along the dissolution pathway 17 having the cross-sectional arc shapes that are formed by the second insoluble unit 16 as shown in FIG. 10(b). When the soluble unit 13 between the insoluble unit 12 and the second insoluble unit 16 completely dissolves, the insoluble unit 12 of the lumen passability checking capsule 20B of which dome-shaped capsule shape has been maintained by the existence of the soluble unit 13 cannot maintain the dome-shaped capsule shape and are reliably separated into the sheet-like members 12a. Subsequently, the soluble unit 13 that fills the opening in the second insoluble unit 16 starts to dissolve, and when that soluble unit 13 dissolves, the second insoluble unit 16 is separated into the sheet-like members 16a.

Because the separated sheet-like members 12a and 16a are formed of elastic members that curl due to its elasticity, the sheet-like members 12a and 16a deform from a flat-sheet shape shown in FIG. 11(a) to a forcibly curled shape shown in FIG. 11(b) and become smaller, and thus easily pass through the stenosis part. In the lumen passability checking capsule 20B in accordance with the fifth variation, the dissolution pathway 17 is formed by the second insoluble unit 16 in a manner such that it takes more time for the soluble unit 13 to dissolve. The dissolution time of the soluble unit 13 can be structurally extended to three days or more, for example, whereby the dome-shaped capsule shape is maintained for a long period of time.

### (Sixth variation)

FIG. 12 is a central cross-sectional side view of a lumen passability checking capsule 20C in accordance with a sixth variation. In the lumen passability checking capsule 20C in accordance with the sixth variation, the insoluble unit 12 is formed in a swirl in a manner such that a portion (e.g., a half circumference) of the insoluble unit 12 overlaps with another portion of the insoluble unit 12 substantially in parallel and in layers via the sheet-like soluble unit 13. A portion of the soluble unit 13 exposed on the surface layer is arranged in a linear manner along the longitudinal axis of the dome-shaped capsule shape. The inner portion of the insoluble unit 12 separates the soluble unit (the soluble unit 13 from the second soluble unit 14) and configures a second insoluble unit 18 that makes the dissolution pathway 17 by the soluble unit 13 to extend along the circumferential direction and to have a cross-sectional arc shape.

When the lumen passability checking capsule 20C in accordance with the sixth variation stagnates in the small intestine, the exposed potion of the soluble unit 13 linearly arranged on the surface layer dissolves first. Then, the dissolution of the soluble unit 13 advances along the dissolution pathway 17 having the cross-sectional arc shape toward the inside. When the entire soluble unit 13 in the dissolution pathway 17 dissolves, the inside second soluble unit 14 dissolves in a short period of time so that the insoluble unit 12 becomes hollow. Then, the insoluble unit 12 of the lumen passability checking capsule 20C of which dome-shaped capsule shape has been maintained by the existence of the soluble unit 13 cannot maintain the dome-shaped capsule shape and, for example, curls and becomes smaller, and thus easily passes through the stenosis part. In the lumen passability checking capsule 20C in accordance with the sixth variation, the dissolution pathway 17 is formed in a manner such that it takes more time for the soluble unit 13 to dissolve using the second insoluble unit 16. Thus, the dissolution time of the soluble unit 13 can be structurally extended, whereby the dome-shaped capsule shape can be maintained for a long period of time.

### (Third example)

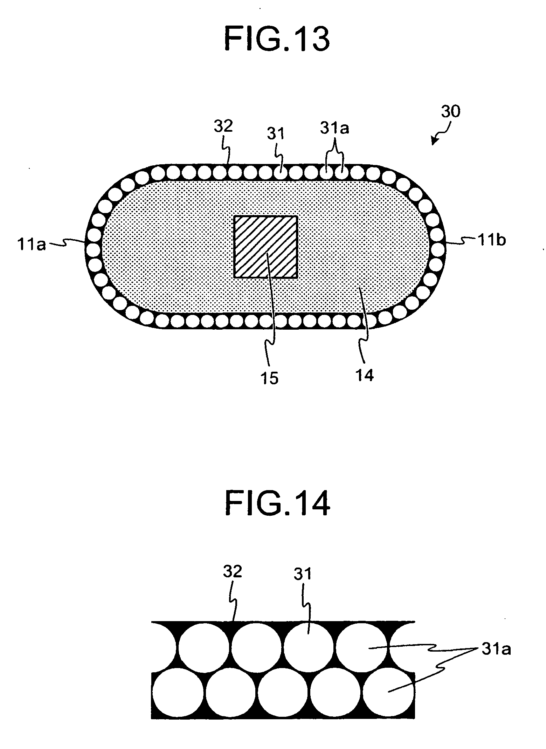

A lumen passability checking capsule in accordance with a third example of the present invention is described with reference to FIG. 13. FIG. 13 is a central vertical cross-sectional front view showing an exemplary configuration of a lumen passability checking capsule 30. The lumen passability checking capsule 30 in accordance with the third example includes an insoluble unit 31 made of a material that does not dissolve in the body and formed of a many number of tiny balls 31a. The tiny balls 31a are connected and linked together by a soluble unit 32 made of a material that dissolves in the body in a thin-wall state to form the surface layer of the dome-shaped capsule shape. The soluble unit 32 is arranged linearly along any directions corresponding to the arrangement of the insoluble unit 31 and therefore is arranged along the longitudinal direction, circumferential direction around the longitudinal axis, and on the convex parts 11a and 11b of the dome-shaped capsule shape. The materials of the insoluble unit 31 and the soluble unit 32 can be the same as those described in the first and second examples. The insoluble unit 31 contains the second soluble unit 14 and the detectable unit 15 as needed.

When the lumen passability checking capsule 30 in accordance with the third example stagnates in the small intestine, the soluble unit 32 exposed to the surface layer dissolves first. Because the soluble unit 32 fills the space among the tiny balls 31a and therefore is partially arranged, only a small part of the soluble unit 32 touches the bodily fluid and it takes a long time for the soluble unit 32 to dissolve. Thus, this structure allows the dome-shaped capsule shape to be maintained for a long time. Even when a portion of the soluble unit 32 among the tiny balls 31a dissolves more quickly than another part of the soluble unit 32 and causes some of the tiny balls 31a to be separated from the surface layer to fall off, the remaining tiny balls 31a that are still linked together by the soluble unit 32 maintain the dome-shaped capsule shape. In that case, although there may be concave or convex portions on the dome-shaped capsule shape, the dome-shaped capsule shape can be structurally maintained for a long time. When most of the soluble unit 32 dissolves, the insoluble unit 31 of which dome-shaped capsule shape has been maintained by the soluble unit 32 becomes smaller because the insoluble unit 31 loses the link among the tiny balls 31a and is separated into each of the tiny balls 31a, i.e., deforms to be smaller. Each of the separated tiny balls 31a can easily passes through the stenosis part. When a stool excreted by the subject contains the tiny balls 31a, such a stool indicates that the lumen passability checking capsule 30 has stagnated in the lumens for more than a predetermined time and thus the stagnation check using X-rays can be eliminated.

### (Seventh variation)

FIG. 14 is a partial cross-sectional view of a lumen passability checking capsule 30A in accordance with a seventh variation. In the lumen passability checking capsule 30A in accordance with the seventh variation, the insoluble unit 31 includes multiple layers, e.g., two layers, of the many number of tiny balls 31a that are linked together by the soluble unit 32. When the lumen passability checking capsule 30A in accordance with the seventh variation stagnates in the small intestine and the dissolution of the soluble unit 32 advances, even when the soluble unit 32 in the surface layer dissolves, the soluble unit 32 in the lower layer keeps the tiny balls 31a of the insoluble unit 31 in the surface layer being linked together. Therefore, the dome-shaped capsule shape can be structurally maintained for a long time. When the lower layer of the soluble unit 32 dissolves, the tiny balls 31a in the surface layer are separated into each of the tiny balls 31a, and the dome-shaped capsule shape becomes smaller. When the remaining soluble unit 32 dissolves, the tiny balls 31a in the lower layer are also separated into each of the tiny balls 31a, and the dome-shaped capsule shape deforms to be smaller. Although FIG. 14 illustrates two layers as exemplary multiple layers, the number of layers can be any number. Furthermore, the tiny ball 31a in each layer can be of any size and the ratio of the tiny balls 31a to the soluble unit 32 can be appropriately changed in accordance with the desired dissolution time.

### (Fourth example)

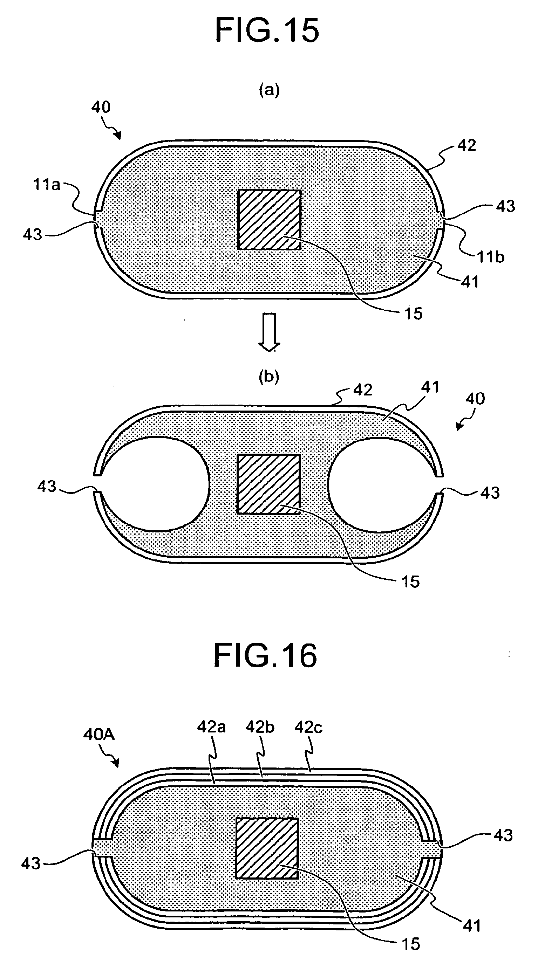

A lumen passability checking capsule in accordance with a fourth example of the present invention is described with reference to FIG. 15. FIG. 15 is a vertical cross-sectional front view of a lumen passability checking capsule 40 in accordance with the fourth example. The lumen passability checking capsule 40 includes a soluble unit 41, an insoluble unit 42, and the detectable unit 15.

The soluble unit 41 is made of a material that dissolves in the body and forms a dome-shaped capsule shaped structure. The material of the soluble unit 41 is an enteric material similarly to those of the above-mentioned embodiments. The insoluble unit 42 is fragile and lacks mechanical strength so that the insoluble unit 42 itself cannot maintain its shape. The insoluble unit 42 is made of a material that does not dissolve in the body basically. For example, the insoluble unit 42 is a metallic thin film made of gold and covers the surface of the soluble unit 41 having the capsule-shaped structure while exposing a part of the soluble unit 41 to the surface through an opening 43. The openings 43 are arranged in the middle of the convex parts 11a and 11b that are arranged at both ends along the longitudinal axis. The insoluble unit 42 is fixed to the soluble unit 41 and therefore the insoluble unit 42 can maintain its capsule shape.

When the lumen passability checking capsule 40 configured as above stagnates in the small intestine, the dissolution of the soluble unit 41 gradually proceeds from a potion of the soluble unit 41 that is exposed to the outside through the opening 43 toward the inside of the soluble unit 41 as shown in FIG. 15(b). While the soluble unit 41 dissolves, the insoluble unit 42 of the surface layer and the inside soluble unit 41 integrally maintain the dome-shaped capsule shape. When elapsing a predetermined time and most of the soluble unit 41 dissolves, the lumen passability checking capsule 40 becomes hollow and the soluble unit 41 cannot maintain the dome-shaped capsule shape and collapses. Furthermore, the insoluble unit 42 that is a thin film cannot maintain the dome-shaped capsule shape and collapses into pieces almost at the same time with the collapse of the soluble unit 41, and thus easily passes through the stenosis part.

A method of manufacturing the lumen passability checking capsule 40 in accordance with the fourth example is described. A first example of the method includes steps of forming the soluble unit 41 that dissolves in the body and has a dome-shaped capsule-shaped structure, and then forming an insoluble unit 42 that does not dissolve in the body and is a thin film that covers the soluble unit 41 excluding a portion of the soluble unit 41 (the opening 43). The insoluble unit 42, which is the thin film, is fixed to the surface of the soluble unit 41.

A second example of the method includes steps of forming the soluble unit 41 that dissolves in the body and has the dome-shaped capsule-shaped structure, then forming the insoluble unit 42 that does not dissolve in the body and is a thin film that is fixed to the whole surface of the structure made of the soluble unit 41, and then removing a portion (the opening 43) of the thin film made of the insoluble unit 42.

The step of forming the insoluble unit 42 that is the thin film that covers the surface of the structure made of the soluble unit 41 can adopt a method of evaporating the material of the insoluble unit 42 or a method of spraying the material and causing the material to harden. Furthermore, the step can adopt a method of using a liquid material of the insoluble unit 42, soaking the structure made of the soluble unit 41 in the liquid material, then taking the structure out of the liquid material, and causing the liquid material to harden. The step in the first example of forming the insoluble unit 42 that is the thin film and covers the structure made of the soluble unit 41 excluding a portion of the soluble unit 41 may adopt a method of masking the surface of the structure made of the soluble unit 41, removing the masked portion after the thin film is formed on the surface so that the thin film made of the insoluble unit 42 can be formed on the portion that has not been masked. The detectable unit 15 may be made of barium or metals that are radiopaque. The insoluble unit 42 or the soluble unit 41 may be radiopaque and function as the detectable unit 15. In this case, the structure is simplified and the production becomes easy. Further, the dissolving state can be confirmed using X-rays.

### (Eighth variation)

FIG. 16 is a vertical cross-sectional front view of a lumen passability checking capsule 40A in accordance with an eighth variation. In the lumen passability checking capsule 40A in accordance with the eighth variation, multiple layers of thin films made of the insoluble unit 42, e.g., three layers of the thin films 42a, 42b, and 42c, are formed on the surface of the structure made of the soluble unit 41. Even when the surface of the lumen passability checking capsule 40A in accordance with the eighth variation is damaged before swallowing or in the lumens, the multiple layers of the insoluble units 42a, 42b, and 42c prevent the inside soluble unit 41 from being exposed. Thus, the soluble unit 41 does not start dissolving from other portions than the opening 43, whereby shortening of the time for which the dome-shaped capsule shape can be maintained is prevented.

A method of manufacturing the lumen passability checking capsule 40A in accordance with the eighth variation includes steps of forming a dome-shaped capsule-shaped structure made of the soluble unit 41, and then repeatedly forming a thin film made of the insoluble unit 42 that does not dissolve in the body and fixing the thin film to the structure made of the soluble unit 41 excluding a portion thereof (the opening 43). In the step of forming the dome-shaped capsule-shaped structure made of the soluble unit 41, the structure is first a little smaller than the desirable size of the dome-shaped capsule shape so that the desirable size of the lumen passability checking capsule 40A can be manufactured at last. In the step of forming the insoluble unit 42 on the surface of the soluble unit 41, the insoluble unit 42 is fixed to the soluble unit 41. In forming the insoluble unit 42 on another insoluble unit 42, the insoluble unit 42 is fixed to another insoluble unit 42.

In the lumen passability checking capsules 40 and 40A in accordance with the fourth example and the eighth variation, the soluble unit 41 has the dome-shaped capsule-shaped structure. Not limited to this, the insoluble unit 42 may arranged in a thin layer to be flexible and have the dome-shaped capsule-shaped structure. In this case, the insoluble unit 42 crushes and becomes smaller after the soluble unit 41 dissolves.

### (Second embodiment)

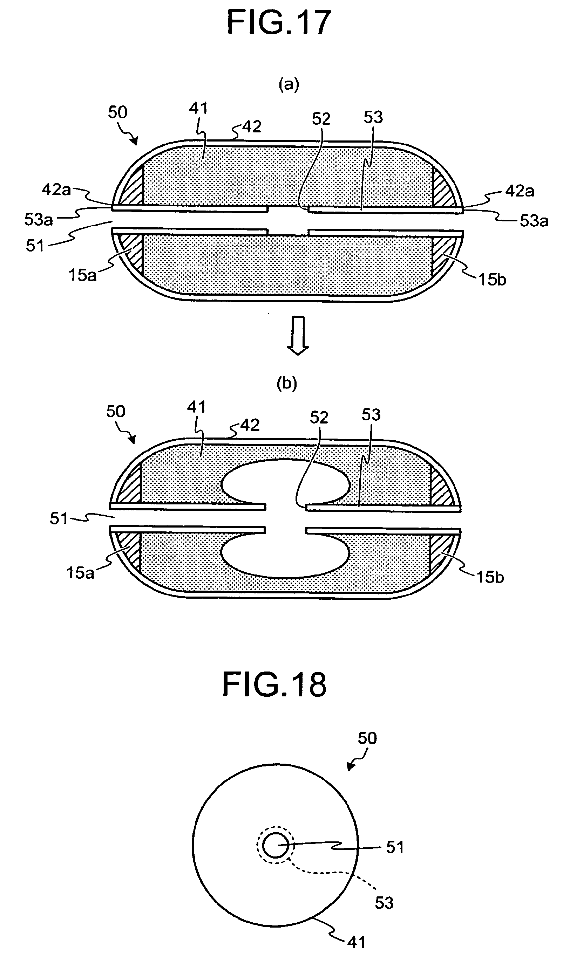

A lumen passability checking capsule in accordance with a second embodiment of the present invention is described with reference to FIGS. 17 and 18. FIG. 17 is a vertical cross-sectional front view showing an exemplary configuration of a lumen passability checking capsule 50 in accordance with the second embodiment, and FIG. 18 is a side view thereof.

In the lumen passability checking capsule 50 in accordance with the second embodiment, a penetration hole 51 is arranged along the central axis in the longitudinal direction of the dome-shaped capsule-shaped structure made of the soluble unit 41. A second insoluble unit 53 covers the inner surface of the soluble unit 41 except for an opening 52 that is located in the penetration hole 51. For example, the opening 52 is located in the center of the longitudinal axis in the second embodiment. The second insoluble unit 53 is made of a material that does not dissolve in the body. An end 53a of the second insoluble unit 53 in the longitudinal direction is integrally connected to an end 42a of the insoluble unit 42 on the surface of the dome-shaped capsule shape. The material of the second insoluble unit 53 may be the same as that of the insoluble unit 42 and may be different. The detectable unit 15 to be provided in the lumen passability checking capsule 50 is divided into detectable units 15a and 15b. The detectable units 15a and 15b are separated from each other and located in both ends in the longitudinal direction at the most distant parts from the opening 52 in the soluble unit 41. The insoluble units 42 and 53 are fragile and lack mechanical strength. The insoluble units 42 and 53 are fixed to the soluble unit 41.

When the lumen passability checking capsule 50 configured as above stagnates in the small intestine, the dissolution of the soluble unit 41 proceeds from a portion of the soluble unit 41 that is exposed to the outside through the opening 52, which is located in the middle in the penetration hole 51, then toward the inside of the soluble unit 41, as shown in FIG. 17(b). Meanwhile, the inside soluble unit 41 allows the insoluble unit 42 of the surface layer to maintain its dome-shaped capsule shape. When elapsing a predetermined time and most of the inside soluble unit 41 dissolves, the lumen passability checking capsule 50 becomes hollow and cannot maintain the dome-shaped capsule shape and collapses. The insoluble unit 42, which is the thin film, also cannot maintain the dome-shaped capsule shape and collapses substantially at the same time with the collapse of the soluble unit 41, whereby the insoluble unit 42 can pass through the stenosis part. When the soluble unit 41, the insoluble unit 42, and the second insoluble unit 53 collapse, the detectable units 15a and 15b are also broken into pieces and pass through the stenosis part and excreted.

In the lumen passability checking capsule 50 in accordance with the second embodiment, the dissolution of the soluble unit 41 starts at the opening 52 that is located in the middle in the penetration hole 51. Because the soluble unit 41 at the opening 52 matters little for maintaining the dome-shaped capsule shape, this structure can maintain the capsule shape for a long period of time. Furthermore, because the end 42a of the insoluble unit 42 is connected to the end 53a of the second insoluble unit 53, the soluble unit 41 overall are continuously protected from being exposed. Therefore, the dissolution of the soluble unit 41 does not start near the end 42a of the insoluble unit 42. Because such a dissolution that can cause the capsule shape to collapse is prevented, this structure can maintain the capsule shape for a long period of time.

In the lumen passability checking capsule 50 in accordance with the second embodiment, the detectable unit 15 is divided into the detectable units 15a and 15b, which are separated from each other. After a predetermined period of time, the state of the capsule can be detected using a detecting apparatus that can detect the positions of the detectable units 15a and 15b. When the detected detectable units 15a and 15b are broken into pieces, it can be determined that the lumen passability checking capsule 50 has collapsed in the lumens. When the detected detectable units 15a and 15b remain unchanged and separate from each other, it can be determined that the lumen passability checking capsule 50 has not collapsed in the lumens and is stagnating in the lumens. Because the detectable units 15a and 15b are located at the positions that are the most distant from the opening 52, the detectable units 15a and 15b can maintain its relative positions until the lumen passability checking capsule 50 completely collapses. Therefore, the collapse of the lumen passability checking capsule 50 can be detected accurately. The detectable unit may be made of metals that are radiopaque and do not dissolve in the intestines or made of barium that dissolves in the intestines. Similarly to the fourth embodiment, the soluble unit 41, the insoluble unit 42, and the second insoluble unit 53 may be radiopaque and function as the detectable unit 15.

In the lumen passability checking capsule 50 in accordance with the second embodiment, the soluble unit 41 has the dome-shaped capsule-shaped structure. Not limited to this, the insoluble unit 42 may arranged in a thin layer to be flexible and have the dome-shaped capsule-shaped structure. In this case, the insoluble unit 42 crushes and becomes smaller after the soluble unit 41 dissolves.

### (Third embodiment)

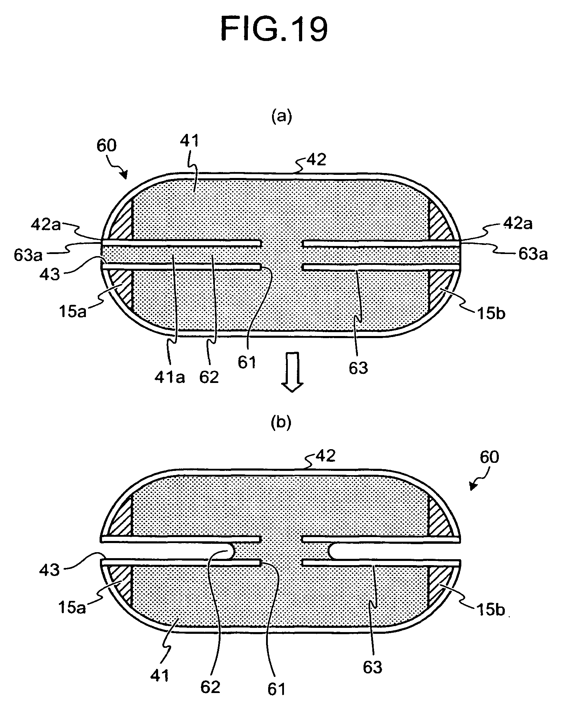

A lumen passability checking capsule in accordance with a third embodiment of the present invention is described with reference to FIG. 19. FIG. 19 is a vertical cross-sectional front view showing an exemplary configuration of the lumen passability checking capsule 60 in accordance with the third embodiment.

The lumen passability checking capsule 60 in accordance with the third embodiment includes a second insoluble unit 63 that forms a dissolution pathway 62. The dissolution pathway 62 is arranged along the longitudinal axis and extended along the soluble unit 41 that has the dome-shaped capsule-shaped structure. The dissolution pathway 62 cylindrically divides the soluble unit 41 along the longitudinal axis and extends from an opening 43 that is a part of the surface of the soluble unit 41 exposed to the outside to through an opening 61 to the inside of the soluble unit 41. The second insoluble unit 63 is made of a material that does not dissolve in the body. An end 63a of the second insoluble unit 63 in the longitudinal direction is integrally connected to the end 42a of the insoluble unit 42 on the surface of the dome-shaped capsule shape. The material of the second insoluble unit 63 may be the same as that of the insoluble unit 42 or may be different. The detectable unit 15 to be provided in the lumen passability checking capsule 60 is divided into the detectable units 15a and 15b, which are separated from each other and located at the deepest parts of the dissolution pathway 62 in the soluble unit 41. The insoluble unit 42 and the second insoluble unit 63 are fragile and lack mechanical strength. The insoluble unit 42 and the second insoluble unit 63 are fixed to the soluble unit 41.

The lumen passability checking capsule 60 in accordance with the third embodiment is manufactured by forming the second insoluble unit 63 on the surface of a cylindrically-formed soluble unit 41a except for the openings 43 and 61, then forming a soluble unit 41 that has the desirable dome-shaped capsule structure, and forming the insoluble unit 42 that is the thin film and covers and fixed to the surface of the structure.

When the lumen passability checking capsule 60 configured as above stagnates in the small intestine, the soluble unit 41 exposed through the opening 43 to the outside dissolves first, and then the dissolution of the soluble unit 41 in the second insoluble unit 63 proceeds along the dissolution pathway 62 as shown in FIG. 19(b). The dissolution then proceeds through the opening 61 to the outside of the second insoluble unit 63. Meanwhile, the inside soluble unit 41 allows the insoluble unit 42 of the surface layer to maintain its dome-shaped capsule shape. When elapsing a predetermined time and most of the inside soluble unit 41 dissolves and the lumen passability checking capsule 60 becomes hollow, the soluble unit 41 cannot maintain the dome-shaped capsule shape and collapses. The insoluble unit 42, which is the thin film, also cannot maintain the dome-shaped capsule shape and collapses substantially at the same time with the collapse of the soluble unit 41, whereby the insoluble unit 42 can pass through the stenosis part. When the soluble unit 41, the insoluble unit 42, and the second insoluble unit 63 collapse, the detectable units 15a and 15b are also broken into pieces and pass through the stenosis part and excreted.

In the lumen passability checking capsule 60 in accordance with the third embodiment, the second insoluble unit 63 divides the soluble unit 41, and the dissolution pathway 62 is arranged from the portion of the surface to the inside of the soluble unit 41. The dissolution time of the soluble unit 41 in the lumens can be structurally lengthened to three days or more, for example, by extending the dissolution pathway 62, so that the capsule shape can be maintained for a long period of time. Thus, the capsule shape can be reliably maintained for a time required for checking the lumen passability. Because the second insoluble unit 63 that forms the dissolution pathway 62 is arranged in a manner such that the second insoluble unit 63 extends to the center of the dome-shaped capsule shape, the dissolution pathway 62 allows only a portion of the soluble unit 41 that matters little for maintaining the dome-shaped capsule shape to dissolve first. The middle of the soluble unit 41 dissolves and then the outer portion of the soluble unit 41 dissolves. Therefore, it takes a long time for the soluble unit 41 near the insoluble unit 42, which is the outer surface of the dome-shaped capsule shape, to dissolve. Since the end 42a of the insoluble unit 42 and the end 63a of the second insoluble unit 63 are connected together and the continuous cover is achieved, the collapse due to the dissolution of the soluble unit 41 does not start near the end 42a of the insoluble unit 42. Therefore, the capsule shape can be structurally maintained for a long time. Furthermore, because the dissolution pathway 62 allows maintaining the capsule shape for a long time, options for choosing materials used for the soluble unit 41 is increased, whereby the simplification of the production and the reduction of the cost can be achieved.

In the lumen passability checking capsule 60 in accordance with the third embodiment, the detectable unit 15 is divided into the detectable units 15a and 15b, which are separated from each other, similarly to the second embodiment. After elapsing a predetermined time, the state of the capsule can be detected using a detecting apparatus that can detect the existence of the detectable units 15a and 15b. When the detected detectable units 15a and 15b are broken into pieces, it can be determined that the lumen passability checking capsule 60 has collapsed in the lumens. When the detected detectable units 15a and 15b remain unchanged and separate from each other, it can be determined that the lumen passability checking capsule 60 has not collapsed in the lumens and is stagnating in the lumens. Because the detectable units 15a and 15b are located at the positions that are the deepest in the dissolution pathway 62, the detectable units 15a and 15b can maintain its relative positions until the lumen passability checking capsule 60 completely collapses. Therefore, the collapse of the lumen passability checking capsule 60 can be detected accurately. The detectable unit may be made of metals that are radiopaque and do not dissolve in the intestines or made of barium, which can dissolve in the intestines. Similarly to the fourth example, the soluble unit 41, the insoluble unit 42, and the second insoluble unit 63 may be radiopaque and function as the detectable unit 15.

### (Ninth variation)

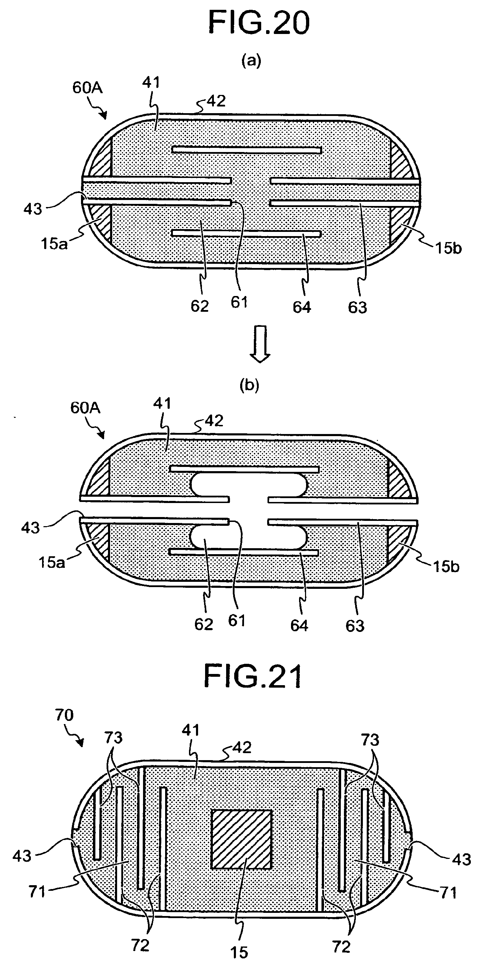

FIG. 20 is a vertical cross-sectional front view of a lumen passability checking capsule 60A in accordance with the ninth variation. The lumen passability checking capsule 60A in accordance with the ninth variation includes a third insoluble unit 64 that has a cylinder shape and made of a material that does not dissolve in the body. The third soluble unit 64 is arranged in the soluble unit 41 on the outer side of the cylindrical second insoluble unit 63. In this way, the dissolution pathway 62 is further divided into smaller portions. The third insoluble unit 64 is located in the middle and surrounds the opening 61. The dissolution pathway 62 proceeds in the following order: the opening 43, the inside of the second insoluble unit 63, the opening 61, the space between the second insoluble unit 63 and the third insoluble unit 64, and the space between the third insoluble unit 64 and the insoluble unit 42. In the lumen passability checking capsule 60 in accordance with the ninth variation, the dissolution pathway 62 is divided into smaller portions and is further lengthened. This structure allows the dissolution time of the soluble unit 41 to be a longer, and thus the dome-shaped capsule shape can be maintained for a longer period of time. Furthermore, because the dissolution pathway 62 allows maintaining the capsule shape for a long time, options for choosing materials used for the soluble unit 41 is increased, whereby the simplification of the production and the reduction of the cost can be achieved.

In the lumen passability checking capsules 60 and 60A in accordance with the third embodiment and the ninth variation, the soluble unit 41 has the dome-shaped capsule-shaped structure. Not limited to this, the insoluble unit 42 may arranged in a thin layer to be flexible and have the dome-shaped capsule-shaped structure. In this case, the insoluble unit 42 crushes and becomes smaller after the soluble unit 41 dissolves.

### (Fourth embodiment)

A lumen passability checking capsule in accordance with a fourth embodiment of the present invention is described with reference to FIG. 21. FIG. 21 is a vertical cross-sectional front view showing an exemplary configuration of a lumen passability checking capsule 70 in accordance with the fourth embodiment.

The lumen passability checking capsule 70 in accordance with the fourth embodiment includes a second insoluble unit 72 that are arranged like a comb and forms a dissolution pathway 71. The dissolution pathways 71 are provided at both ends in the longitudinal direction of the dome-shaped capsule shape. The dissolution pathway 71 proceeds from the opening 43 toward the inner-central side and divides the soluble unit 41 in a direction crossing the longitudinal axis. Furthermore, the lumen passability checking capsule 70 includes a third insoluble unit 73 that is arranged like a comb. The third insoluble unit 73 divides the soluble unit 41 in a direction crossing the longitudinal axis from the opposite side to the second insoluble unit 72. In this way, the dissolution pathway 71 is further divided into smaller portions. The second insoluble unit 72 and the third insoluble unit 73 are made of materials that do not dissolve in the body and are arranged like combs that mesh to each other, so that the dissolution pathway 71 is a prolonged like a maze that starts at the opening 43 and turns. The insoluble unit 42, the second insoluble unit 72, and the third insoluble unit 73 are fragile and lack mechanical strength. The insoluble unit 42, the second insoluble unit 72, and the third insoluble unit 73 are fixed to the soluble unit 41.

When the lumen passability checking capsule 70 configured as above stagnates in the small intestine, a portion of the soluble unit 41 that is exposed through the opening 43 to the outside dissolves first and the dissolution of the soluble unit 41 proceeds to the inside of the soluble unit 41 along the dissolution pathway 71, which is like a maze. Meanwhile, the inside soluble unit 41 allows the insoluble unit 42 of the surface layer to maintain its dome-shaped capsule shape. When elapsing a predetermined time and most of the inside soluble unit 41 dissolves and becomes hollow, the soluble unit 41 cannot maintain the dome-shaped capsule shape and collapses. The insoluble unit 42, which is the thin film, also cannot maintain the dome-shaped capsule shape and collapses substantially at the same time with the collapse of the soluble unit 41, whereby the insoluble unit 42 can pass through the stenosis part.

In the lumen passability checking capsule 70 in accordance with the fourth embodiment, the second insoluble unit 72 and the third insoluble unit 73 divides the dissolution pathway 71 into smaller portions that are filled with the soluble unit 41. The dissolution pathway 71 is a long pathway, and this structure allows the dissolution time of the soluble unit 41 to be lengthened so that the capsule shape can be maintained for a long period of time. Thus, the capsule shape can be reliably maintained for a time required for checking the passability, and the lumen passability can be reliably checked. The detectable unit may be made of metals that are radiopaque and do not dissolve in the intestines or made of barium that can dissolve in the intestines. Similarly to the fourth example, the soluble unit 41, the insoluble unit 42, the second insoluble unit 72, and the third insoluble unit 73 may be radiopaque and may function as the detectable unit 15.

### (Fifth embodiment)

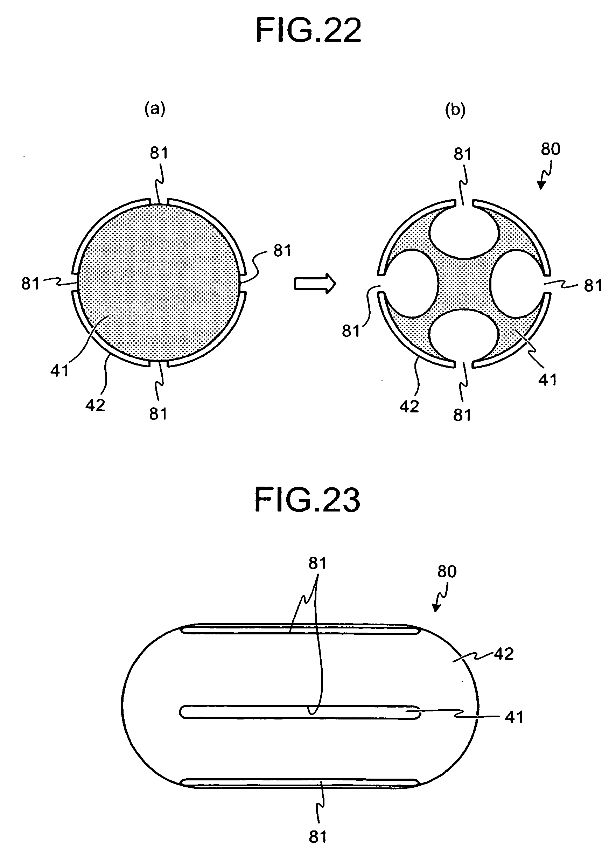

A lumen passability checking capsule in accordance with an fifth embodiment of the present invention is described with reference to FIGS. 22 and 23. FIG. 22 is a central vertical cross-sectional side view showing an exemplary configuration of a lumen passability checking capsule 80 in accordance with the fifth embodiment, and FIG. 23 is a front view thereof.

The lumen passability checking capsule 80 in accordance with the fifth embodiment, has a number of openings 81, e.g., four openings 81, on the surface of the soluble unit 41 that has the dome-shaped capsule-shaped structure. The soluble unit 41 is exposed through the openings 81 to the surface of the insoluble unit 42. The openings 81 are linearly formed along the longitudinal axis.

When the lumen passability checking capsule 80 configured as above stagnates in the small intestine, the dissolution of the soluble unit 41 gradually proceeds from a portion that is exposed through the opening 81 to the surface toward the inside. Meanwhile, the inside soluble unit 41 allows the insoluble unit 42 of the surface layer to maintain the dome-shaped capsule shape. When elapsing a predetermined time and most of the soluble unit 41 dissolves, the insoluble unit 42, which is the thin film, cannot maintain its dome-shaped capsule shape and collapse into small pieces or crushes, whereby the insoluble unit 42 can pass through the stenosis part.

### (Tenth variation)

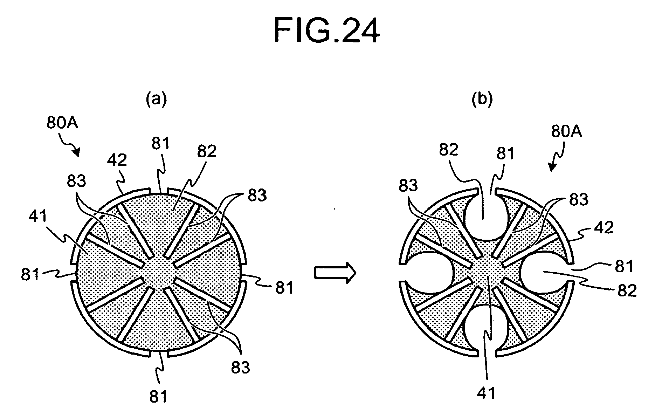

FIG. 24 is a central vertical cross-sectional side view of a lumen passability checking capsule 80A in accordance with a tenth variation. The lumen passability checking capsule 80A in accordance with the tenth variation includes a second insoluble unit 83 that is arranged radially and forms a dissolution pathway 82 on an inner circumferential side of the insoluble unit 42. The dissolution pathway 82 is arranged from the opening 81 to the inside of the soluble unit 41. The dissolution pathway 82 divides the soluble unit 41 along the radial direction inside the insoluble unit 42. The dissolution pathway 82, which is formed by the second insoluble unit 83, proceeds from the opening 81 toward the shaft center, and then toward the inner surface of the insoluble unit 42 while making a turn in the radial direction.

When the lumen passability checking capsule 80A configured as above stagnates in the small intestine, the dissolution of the soluble unit 41 begins at the portion that is exposed through the opening 81 to the outside. Then, the dissolution of the soluble unit 41 proceeds along the dissolution pathway 82 formed by the second insoluble unit 83 to the inside, specifically, toward the shaft center. Meanwhile, the inside soluble unit 41 allows the insoluble unit 42 of the surface layer to maintain the dome-shaped capsule shape. When elapsing a predetermined time and the soluble unit 41 on the inner surface of the insoluble unit 42 dissolves, the insoluble unit 42, which is the thin film, cannot maintain the dome-shaped capsule shape and is broken into pieces or crushes, whereby the insoluble unit 42 can pass through the stenosis part.

The lumen passability checking capsule 80A in accordance with the tenth variation includes the dissolution pathway 82 that is formed in the soluble unit 41 by the second insoluble unit 83. The dissolution pathway 82 is divided by the second insoluble unit 83, and this structure allows the dissolution time of the soluble unit 41 to be prolonged, and thus the capsule shape can be maintained for a long time. The structure can reliably maintain the capsule shape for a time required for checking the passability, and thus the lumen passability can be reliably checked.

In the lumen passability checking capsules 80 and 80A in accordance with the fifth embodiment and the tenth variation, the soluble unit 41 has the dome-shaped capsule-shaped structure. Not limited to this, the insoluble unit 42 may arranged in a thin layer to be flexible and have the dome-shaped capsule-shaped structure. In this case, the insoluble unit 42 crushes and becomes smaller after the soluble unit 41 dissolves.
For example, in the embodiments and variations described above, the lumen passability checking capsule has the dome-shaped capsule shape and includes the dome-shaped convex parts 11a and 11b at the both ends in the longitudinal direction thereof. Not limited to this, the lumen passability checking capsule maybe a spherical-shaped capsule shape whose diameter is substantially the same as a diameter of the capsule endoscope along the short axis thereof. Furthermore, the insoluble unit is made of materials that do not basically dissolve inside the body. Not limited to this, the insoluble unit may be made of a material that is hard to dissolve in the body relative to the soluble unit. In this case, the material for the insoluble unit may be one that dissolves in the body but takes a longer time compare to the material of the soluble unit (i.e., the material harder to dissolve).

### INDUSTRIAL APPLICABILITY

The lumen passability checking device and the methods of dissolving and manufacturing the device in accordance with the present invention are suitable for checking beforehand the passability through the lumens and organs of a body of a subject before actually using a capsule medical device, and are particularly applicable to a case in which a small intestine is being considered as the lumens and organs.

## Claims

1. A lumen passability checking device (10; 10A; 10B; 10C; 20; 20A; 20B; 20C; 30; 40; 40A; 50; 60; 60A; 70; 80; 80A) having a capsule shape and a size insertable into a body, the lumen passability checking device comprising:
a soluble unit (13, 14; 32, 14; 41) made of a material that dissolves in the body and forming a structure of the capsule shape; and
an insoluble unit (12; 31; 42) made of a material that does not dissolve in the body and forming a thin film that covers a surface of the soluble unit excluding an opening,
wherein the shape of the insoluble unit (12; 31; 42) is maintained by the soluble unit (13, 14; 32, 14; 41), and the insoluble unit (12; 31; 42) collapses when the soluble unit (13, 14; 32, 14; 41) dissolves and collapses, **characterized by** a second insoluble unit (18; 53; 63; 72; 83) made of a material that does not dissolve in the body, said second insoluble unit forming a dissolution pathway (17; 62; 71; 82) that divides inside the soluble unit and extends from the opening to the inside.

2. The lumen passability checking device (50; 60; 80A) according to claim 1, wherein the second insoluble unit (53; 63; 83) extends along a direction toward a center of the capsule shape.

3. The lumen passability checking device (70) according to claim 2, further comprising a third insoluble unit (73) made of a material that does not dissolve in the body, said third insoluble unit further dividing the dissolution pathway (71) into smaller portions.

4. The lumen passability checking device (10; 10A; 10B; 10C; 20; 20A; 20B; 20C; 30; 40; 40A; 50; 60; 60A; 70; 80; 80A) according to claim 1, wherein the insoluble unit is provided in a manner fixed to the soluble unit.

## Patentansprüche

1. Lumenpassierbarkeitsüberprüfungsgerät (10; 10A; 10B; 10C; 20; 20A; 20B; 20C; 30; 40; 40A; 50; 60; 60A; 70; 80; 80A), das eine Kapselform aufweist und eine Größe, die in einen Körper einführbar ist, wobei das Lumenpassierbarkeitsüberprüfungsgerät umfasst:
eine lösliche Einheit (13, 14; 32, 14; 41) aus einem Material, das sich in dem Körper auflöst und eine Struktur der Kapselform bildet; und
eine unlösliche Einheit (12; 31; 42) aus einem Material, das sich nicht in dem Körper auflöst und einen dünnen Film bildet, der eine Oberfläche der löslichen Einheit bis auf eine Öffnung bedeckt,
wobei die Form der unlöslichen Einheit (12; 31; 42) durch die lösliche Einheit (13, 14; 32, 14; 41) beibehalten wird, und die unlösliche Einheit (12; 31; 42) zusammenfällt, wenn die lösliche Einheit (13, 14; 32, 14; 41) sich auflöst und zusammenfällt, **gekennzeichnet durch** eine zweite unlösliche Einheit (18; 53; 63; 72; 83) aus einem Material, das sich in dem Körper nicht auflöst, wobei die zweite unlösliche Einheit einen Auflösungspfad (17; 62; 71; 82) bildet, der das Innere der löslichen Einheit aufteilt und sich von der Öffnung in das Innere erstreckt.

2. Lumenpassierbarkeitsüberprüfungsgerät (50; 60; 80A) nach Anspruch 1, wobei die zweite unlösliche Einheit (53; 63; 83) sich entlang einer Richtung zu einer Mitte der Kapselform erstreckt.

3. Lumenpassierbarkeitsüberprüfungsgerät (70) nach Anspruch 2, ferner umfassend eine dritte unlösliche Einheit (73) aus einem Material, das sich in dem Körper nicht auflöst, wobei die dritte unlösliche Einheit ferner den Auflösungspfad (71) in kleinere Abschnitte aufteilt.

4. Lumenpassierbarkeitsüberprüfungsgerät (10; 10A; 10B; 10C; 20; 20A; 20B; 20C; 30; 40; 40A; 50; 60; 60A; 70; 80; 80A) nach Anspruch 1, wobei die unlösliche Einheit auf eine Art bereitgestellt ist, dass sie an der löslichen Einheit angebracht ist.

## Revendications

1. Dispositif (10 ; 10A ; 10B ; 10C ; 20 ; 20A ; 20B ; 20C ; 30 ; 40 ; 40A ; 50 ; 60 ; 60A ; 70 ; 80 ; 80A) de contrôle de la possibilité de passage d'une lumière présentant une forme de capsule et une taille pouvant être insérée dans un corps, le dispositif de contrôle de la possibilité de passage d'une lumière comprenant :
une unité (13, 14 ; 32, 14 ; 41) soluble constituée d'un matériau qui se dissout dans le corps et formant une structure de la forme d'une capsule ; et
une unité (12 ; 31 ; 42) insoluble constituée d'un matériau qui ne se dissout pas dans le corps et formant un film mince qui couvre une surface de l'unité soluble excluant une ouverture,
dans lequel la forme de l'unité (12 ; 31 ; 42) insoluble est maintenue par l'unité (13, 14 ; 32, 14 ; 41) soluble, et l'unité (12 ; 31 ; 42) insoluble s'effondre lorsque l'unité (13, 14 ; 32, 14 ; 41) soluble se dissout et s'effondre, **caractérisé par** une deuxième unité (18 ; 53 ; 63 ; 72 ; 83) insoluble constituée d'un matériau qui ne se dissout pas dans le corps, ladite deuxième unité insoluble formant une voie de passage (17 ; 60 ; 71 ; 82) de dissolution qui divise l'intérieur de l'unité soluble et s'étend de l'ouverture vers l'intérieur.

2. Dispositif (50 ; 60 ; 80A) de contrôle de la possibilité de passage d'une lumière selon la revendication 1, dans lequel la deuxième unité (53 ; 63 ; 83) insoluble s'étend le long d'une direction vers un centre de la forme de capsule.

3. Dispositif (70) de contrôle de la possibilité de passage d'une lumière selon la revendication 2, comprenant en outre une troisième unité (73) insoluble constituée d'un matériau qui ne se dissout pas dans le corps, ladite troisième unité insoluble divisant en outre la voie de passage (71) de dissolution en parties plus petites.

4. Dispositif (10 ; l0A ; 10B ; 10C ; 20 ; 20A ; 20B ; 20C ; 30 ; 40 ; 40A ; 50 ; 60 ; 60A ; 70 ; 80 ; 80A) de contrôle de la possibilité de passage d'une lumière selon la revendication 1, dans lequel l'unité insoluble est prévue d'une manière fixée à l'unité soluble.
